# EUROPEAN PATENT SPECIFICATION

(11) **EP 4 149 455 B1**
(45) Date of publication and mention of the grant of the patent: **04.03.2026**
(21) Application number: 21725127.1
(22) Date of filing: 10.05.2021
(51) Int. Cl.: A61K 31/337, A61K 31/5377, A61K 45/06, A61P 35/00

(54) **COMBINATION OF THE ATR INHIBITOR AZD6738 AND PACLITAXEL FOR USE IN TREATING MELANOMA IN PATIENTS WHO PREVIOUSLY RECEIVED IMMUNOTHERAPY**
KOMBINATION DES ATR-INHIBITORS AZD6738 UND PACLITAXEL ZUR BEHANDLUNG VON MELANOMEN BEI PATIENTEN, DIE ZUVOR EINE IMMUNTHERAPIE ERHALTEN HABEN
ASSOCIATION DE L'INHIBITEUR DE L'ATR AZD6738 ET DE PACLITAXEL POUR LE TRAITEMENT DU MÉLANOME CHEZ LES PATIENTS AYANT DÉJÀ REÇU UNE IMMUNOTHÉRAPIE

(30) Priority: 11.05.2020 US 202063022730 P; 26.03.2021 US 202163166291 P
(43) Date of publication of application: 22.03.2023
(73) Proprietor: Astrazeneca AB, 151 85 Södertälje (SE)
(72) Inventor: SMITH, Simon, Andrew, Cambridge Cambridgeshire CB2 0AA (GB); DEAN, Emma, Jane, Cambridge Cambridgeshire CB2 0AA (GB); CLACK, Glen, Cambridge Cambridgeshire CB2 0AA (GB); LEE, Jeeyun, Seoul 135-710 (KR)
(74) Representative: AstraZeneca Intellectual Property
(86) International application number: PCT/EP2021/062307
(87) International publication number: WO 2021/228758

(56) References cited:
- WO-A1-2020/064971
- ANONYMOUS: "History of Changes for Study: NCT03780608 Phase II Study of AZD6738 in Combination With Durvalumab in Patients With Solid Tumor", CLINICALTRIALS.GOV, 26 December 2019 (2019-12-26), pages 1 - 6, XP055822558, Retrieved from the Internet <URL:https://clinicaltrials.gov/ct2/history/NCT03780608?V_3=View#StudyPageTop> [retrieved on 20210708]
- ANONYMOUS: "History of Changes for Study: NCT03833440 Precision Immuno-Oncology for Advanced Non-small Cell Lung Cancer Patients With PD-1 ICI Resistance (PIONeeR)", CLINICALTRIALS.GOV, 3 March 2020 (2020-03-03), pages 1 - 6, XP055822586, Retrieved from the Internet <URL:https://www.clinicaltrials.gov/ct2/history/NCT03833440?V_2=View#StudyPageTop> [retrieved on 20210708]
- MEI LIN ET AL: "Ataxia telangiectasia and Rad3-related inhibitors and cancer therapy: where we stand", JOURNAL OF HEMATOLOGY & ONCOLOGY, vol. 12, no. 1, 24 April 2019 (2019-04-24), XP055822352, Retrieved from the Internet <URL:http://link.springer.com/article/10.1186/s13045-019-0733-6/fulltext.html> DOI: 10.1186/s13045-019-0733-6
- ANONYMOUS: "Study of AZD6738, DNA Damage Repair/Novel Anti-cancer Agent, in Combination With Paclitaxel, in Refractory Cancer - Full Text View - ClinicalTrials.gov", CLINICALTRIALS.GOV, 22 January 2020 (2020-01-22), pages 1 - 9, XP055822243, Retrieved from the Internet <URL:https://clinicaltrials.gov/ct2/show/NCT02630199?term=AZD6738,+paclitaxel&draw=2&rank=1> [retrieved on 20210707]
- RI-YAO YANG ET AL: "Inhibition of ATR downregulates PD-L1 and sensitizes tumor cells to T cell- mediated killing PD-1/PD-L1 and cancer immunotherapy View project Immunotherapy for Inflammatory diseases View project", 15 July 2018 (2018-07-15), XP055655743, Retrieved from the Internet <URL:https://www.researchgate.net/profile/Mei_Kuang_Chen/publication/326978184_Inhibition_of_ATR_downregulates_PD-L1_and_sensitizes_tumor_cells_to_T_cell-mediated_killing/links/5c707621458515831f67bb6b/Inhibition-of-ATR-downregulates-PD-L1-and-sensitizes-tumor-cells-to-T-cell-mediated-killing.pdf> [retrieved on 20200108]
- YAP T A ET AL: "Phase I modular study of AZD6738, a novel oral, potent and selective ataxia telangiectasia Rad3-related (ATR) inhibitor in combination (combo) with carboplatin, olaparib or durvalumab in patients (pts) with advanced cancers", EUROPEAN JOURNAL OF CANCER, vol. 69, 30 November 2016 (2016-11-30), XP029843494, ISSN: 0959-8049, DOI: 10.1016/S0959-8049(16)32607-7
- DAVID ESCORS ET AL: "The intracellular signalosome of PD-L1 in cancer cells", SIGNAL TRANSDUCTION AND TARGETED THERAPY, vol. 3, no. 1, 28 September 2018 (2018-09-28), XP055626319, DOI: 10.1038/s41392-018-0022-9
- HERNANDEZ M. ET AL: "P16.07 Immuno-Modulatory Effects of Ceralasertib in Combination with Durvalumab in NSCLC with Progression on Anti-PD(L)1 Treatment (HUDSON)", JOURNAL OF THORACIC ONCOLOGY - 2020 WORLD CONFERENCE ON LUNG CANCER, 1 March 2021 (2021-03-01), pages S350 - S350, XP055822517, Retrieved from the Internet <URL:https://www.jto.org/action/showPdf?pii=S1556-0864(21)00595-5> [retrieved on 20210708], DOI: 10.1016/j.jtho.2021.01.552
- LEE JEEYUN: "Results from a phase I, open-label study of ceralasertib (AZD6738), a novel DNA damage repair agent, in combination with weekly paclitaxel in refractory cancer (NCT02630199). | Journal of Clinical Oncology", DEVELOPMENTAL THERAPEUTICS-MOLECULARLY TARGETED AGENTS AND TUMOR BIOLOGY, 25 May 2020 (2020-05-25), pages 1 - 3, XP055822253, Retrieved from the Internet <URL:https://ascopubs.org/doi/abs/10.1200/JCO.2020.38.15_suppl.3503> [retrieved on 20210707]

## Description

### FIELD

The present specification relates to the ATR inhibitor 4-{4-[(3*R*)-3-methylmorpholin-4-yl]-6-[1-((*R*)-*S*-methylsulfonimidoyl)cyclopropyl]pyrimidin-2-yl}-1H-pyrrolo[2,3-b]pyridine (AZD6738, ceralasertib, Compound (I) below or a pharmaceutically acceptable salt thereof) in combination with paclitaxel for use in the treatment of melanoma, characterised in that the combination is administered to a patient who has previously received immunotherapy comprising treatment with a PD-1 inhibitor or a PD-L1 inhibitor, as defined in the claims.

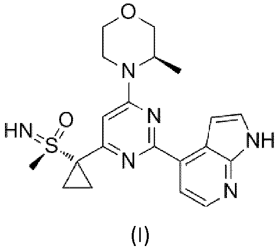

### BACKGROUND

ATR is a serine/threonine protein kinase and member of the phosphatidylinositol 3-kinase related kinase (PIKK) family. During normal DNA replication, ATR is recruited at stalled replication forks, which can progress to double strand breaks if left unrepaired. ATR is also recruited to single strand DNA coated with Replication Protein A (RPA) following single strand DNA damage or the resection of double strand breaks. Recruitment and activation of ATR leads to cell cycle arrest in the S-phase while the DNA is repaired and the stalled replication fork resolved, or nuclear fragmentation and entry into programmed cell death (apoptosis).

As a result, ATR inhibitors are expected to cause growth inhibition in tumour cells dependent upon ATR for DNA repair e.g. ATM-deficient tumours. In addition to such monotherapy activity, ATR inhibitors are also predicted to potentiate the activity of cytotoxic DNA damaging agents and radiotherapy (through inhibition of ATR-dependent DNA repair processes) when used in combination.

Example ATR inhibitors include AZD6738, a potent inhibitor of ATR with good selectivity against other PIKK family members first disclosed in WO2011/154737. This compound is being developed as an oral anti-tumour agent in patients with disease that is dependent upon ATR function for DNA repair, for example tumours that are deficient of the serine/threonine-specific protein kinase, ATM.

ATR inhibitors are being investigated against various forms of cancer, including malignant melanoma. Melanoma develops from the pigment-producing cells known as melanocytes in the skin and is the most dangerous form of skin cancer. In 2015, there were 3.1 million people with active melanoma, which resulted in 59,800 deaths (Vos et al., Lancet 388, 1545-1602); while in 2020 the American Cancer Society estimates about 100,000 new cases will be diagnosed locally, with around 7,000 deaths.

The current standard of care for melanoma is based on first-line immunotherapy, for example using immune checkpoint inhibitors such as nivolumab or pembrolizumab. Patients with actionable mutations, such as those with BRAF mutations may receive targeted agents. Patients may receive several different lines of immunotherapy but once it is no longer effective, standard chemotherapy like doublet carboplatin and paclitaxel or single agent paclitaxel may be used to continue treatment. However, response to chemotherapy is often poor with about 20% responding to doublet chemotherapy and only around 5% of patients responding to taxanes. There is therefore a pressing need for additional approaches that can be used to treat resistant cancers like melanoma which are no longer amenable to immunotherapy.

The phase II clinical trial NCT03780608, available on ClinicalTrials.gov, investigates the use of AZD6738 in combination with durvalumab in patients with solid tumors. The phase 2 clinical trial NCT03833440, also available on ClinicalTrials.gov, assesses how to overcome resistance to immune check point inhibitor (ICI) monotherapies or ICI in combination with platinum-based chemotherapies in patients with advanced non-small cel lung cancer and includes an arm exploring the combination of durvalumab and AZD6738. Mei Lin et al discusses the use of ATR inhibitors in the treatment of cancer and reviews preclinical and clinical data from early-phase trials, either as monotherapy or in combination (Journal of Hematology & Oncology, vol. 12, no. 1, 24 April 2019).

### SUMMARY

The references to methods of treatment in this description are to be interpreted as references to the compounds, pharmaceutical compositions and medicaments of the present invention for use in a method for treatment of the human (or animal) body by therapy.

Surprisingly, it has been found that the ATR inhibitor AZD6738 is particularly effective in melanoma cancer patients who have previously received treatment with immunotherapy comprising treatment with a PD-1 or PD-L1 inhibitor, with a high proportion of such individuals responding to subsequent ATR inhibition. Patients who have received prior immunotherapy have therefore been identified as a target population for treatment with the ATR inhibitor AZD6738. ATR inhibition appears to increase the response rate of immunotherapy-resistant cancers to chemotherapy with the taxane paclitaxel.

Without wishing to be bound by theory, it is believed that cancers treated with immunotherapy may become sensitized to ATR inhibitor treatment, even after the immunotherapy itself ceases to be effective in managing disease.

It is an object of the present specification to provide new uses of the ATR inhibitor AZD6738, particularly in the treatment of melanoma.

In an aspect of the present specification there is provided an ATR inhibitor for use in the treatment of melanoma in a patient, where the treatment comprises the separate, sequential or simultaneous administration of:
a. the ATR inhibitor AZD6738, and
b. paclitaxel,
to said patient, wherein the patient has previously received immunotherapy comprising treatment with a PD-1 or PD-L1 inhibitor.

### FIGURES

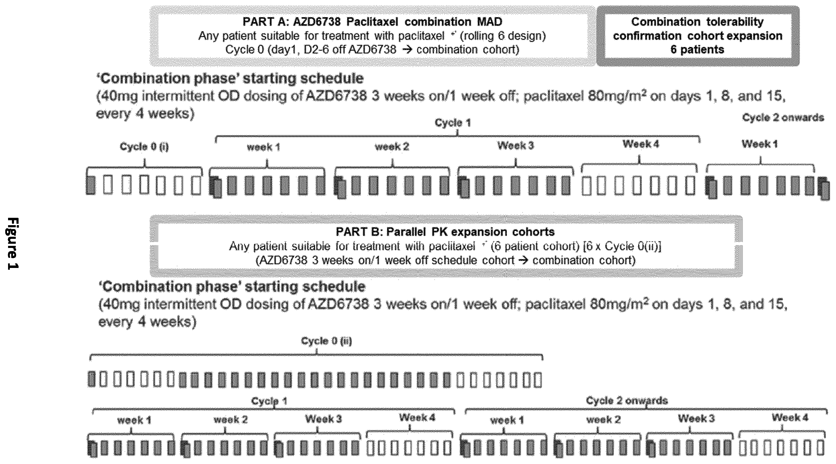

Figure 1: Study design for a clinical trial designed to evaluate the therapeutic potential of AZD6738.
Figure 2: Waterfall plot of best % change in sum of target lesions in the full analysis set of patients receiving AZD6738 and paclitaxel treatment. Y-axis represents % of maximum tumor reduction assessed according to RECIST v1.1 criteria. M = melanoma. The bars are coded by best overall response.
Figure 3: Bar chart showing melanoma patient responses to combined AZD6738 and paclitaxel treatment.
Figure 4: Swimmer plot showing duration of AZD6738 and paclitaxel treatment for melanoma patients along with patient response and ATM mutation status.
Figure 5: Updated swimmer plot showing duration of AZD6738 and paclitaxel treatment for melanoma patients with non-progressive disease (CR, PR or SD) as best response. The bars are coded by best response.

### DETAILED DESCRIPTION

The invention detailed in this specification should not be interpreted as being limited to any of the recited embodiments or examples. Other embodiments will be readily apparent to a reader skilled in the art. The invention is as defined in the claims.

"A" or "an" mean "at least one". In any embodiment where "a" or "an" are used to denote a given element, "a" or "an" may mean one. In any embodiment where "a" or "an" are used to denote a given element, "a" or "an" may mean 1, 2, 3, 4, 5, 6, 7, 8, 9 or 10.

When an embodiment includes "a" or "an" element X, subsequent referrals to "the" element X do not imply only one of the element is present. Instead the above interpretation of "a" or "an" continues to apply so that "the" also means "at least one". In other words, embodiments comprising "an element X, where the element X is..." should be construed as "at least one element X, where the at least one element X is...".

"Comprising" means that a given material or element may contain other materials or elements. In any embodiment where "comprising" is mentioned the given material or element may be formed of at least 10% w/w, at least 20% w/w, at least 30% w/w, or at least 40% w/w of the material or element (or combination of materials or elements). In any embodiment where "comprising" is mentioned, "comprising" may also mean "consisting of" (or "consists of") or "consisting essentially of" (or "consists essentially of") a given material or element.

"Consisting of" or "consists of" means that a given material or element is formed entirely of the material or element (or combination of materials or elements). In any embodiment where "consisting of" or "consists of" is mentioned the given material or element may be formed of 100% w/w of the material or element.

"Consisting essentially of" or "consists essentially of" means that a given material or element consists almost entirely of that material or element (or combination of materials or elements). In any embodiment where "consisting essentially of" or "consists essentially of" is mentioned the given material or element may be formed of at least 50% w/w, at least 60% w/w, at least 70% w/w, at least 80% w/w, at least 90% w/w, at least 95% w/w or at least 99% w/w of the material or element.

In any embodiment where "is" or "may be" is used to define a material or element, "is" or "may be" may mean the material or element "consists of" or "consists essentially of" the material or element.

When it is mentioned that "in some embodiments..." a certain element may be present, the element may be present in a suitable embodiment in any part of the specification, not just a suitable embodiment in the same section or textual region of the specification.

When a feature is "selected from" a particular list, the feature may be selected from a list consisting of the specified alternatives (i.e. a list of those alternatives and no others).

### Therapeutic Use

In one instance of the disclosure, there is provided an ATR inhibitor for use in the treatment of cancer, where the ATR inhibitor is administered to a patient who has previously received immunotherapy.

In one instance there is provided a method of treating cancer in a human or animal patient in need of such treatment, comprising administering an ATR inhibitor to a patient who has previously received immunotherapy.

In one instance there is provided the use of an ATR inhibitor in the manufacture of a medicament for the treatment of cancer, where the medicament is administered to a patient who has previously received immunotherapy.

In one instance there is provided a pharmaceutical composition comprising an ATR inhibitor and a pharmaceutically acceptable excipient for use in the treatment of cancer, where the pharmaceutical composition is administered to a patient who has previously received immunotherapy.

### Immunotherapy

Where a patient has "previously received immunotherapy", this includes patients who have been successfully or unsuccessfully treated with immunotherapy, such that their cancer responded or did not respond to treatment respectively. Patients who have previously received immunotherapy may have halted previous treatment due to treatment failure, where the cancer growth or health impact of the disease is not, or is no longer, positively managed by the immunotherapy. Where such a treatment has failed, the cancer may be described as resistant to immunotherapy. Primary resistance occurs when some inherent characteristic of the cancer prevents the immunotherapy from working whereas acquired resistance, also known as secondary resistance, occurs when the cancer becomes resistant during immunotherapy treatment. Some patients may receive immunotherapy as an adjuvant therapy. Patients who relapse on adjuvant immunotherapy may also be considered to have primary resistance to immunotherapy.

In some embodiments, the patient's cancer may be resistant to immunotherapy.

In some embodiments, the patient has primary resistance to immunotherapy. In one embodiment, primary resistance is defined according to the Society for Immunotherapy of Cancer (SITC) recommendations as having ≥6 weeks of immunotherapy drug exposure and a best response of progressive disease or stable disease for less the 6 months before progressing.

In some embodiments, the patient has acquired resistance to immunotherapy. In one embodiment, acquired resistance is defined according to SITC recommendations as having ≥6 weeks of immunotherapy drug exposure and a best response of complete response, progressive disease or stable disease for more than 6 months before progressing.

"Immunotherapy" is the use of a patient's own immune system to treat disease, for example cancer. It includes stimulating the natural defences of a patient's immune system so it is better at finding and attacking harmful species in the body (for example cancer cells), as well as administering drugs that act like immune system components to restore or improve how the immune system works to defend the body (for example to find and attack cancer cells).

In some instances immunotherapy may comprise treatment with an immune checkpoint inhibitor, chimeric antigen receptor T-cell therapy, treatment with a cytokine, treatment with an immunomodulator, treatment with a cancer vaccine, treatment with a monoclonal antibody and/or treatment with an oncolytic virus.

"Checkpoint inhibitors" include any substance which blocks immune checkpoints: key regulators of the immune system that when stimulated can dampen the immune response to an immunologic stimulus. Some cancers can protect themselves from attack by stimulating immune checkpoint targets, so checkpoint therapy is used that can block inhibitory checkpoints, restoring immune system function.

Example checkpoint inhibitors include PD-1 inhibitors (for example pembrolizumab [Keytruda^{®}], nivolumab [Opdivo^{®}], cemiplimab [Libtayo^{®}], spartalizumab [PDR001], camrelizumab [SHR1210], sintilimab [IBI308], tislelizumab [BGB-A317], toripalimab [JS 001], AMP-224 or AMP-514), PD-L1 inhibitors (for example atezolizumab [Tecentriq^{®}], avelumab [Bavencio^{®}], durvalumab [Imfinzi^{®}], KN035, CK-301, AUNP12, CA-170 or BMS-986189) and CTLA-4 inhibitors (for example ipilimumab [Yervoy^{®}] or tremelimumab).

In some instances immunotherapy may comprise treatment with an immune checkpoint inhibitor.

In some instances immunotherapy may comprise treatment with an immune checkpoint inhibitor selected from a PD-1 inhibitor, a PD-L1 inhibitor and a CTLA-4 inhibitor. According to the invention, previously received immunotherapy comprises treatment with a PD-1 inhibitor or a PD-L1 inhibitor.

In some of the claimed embodiments immunotherapy may comprise treatment with an immune checkpoint inhibitor which is a PD-1 inhibitor.

In some of the claimed embodiments immunotherapy may comprise treatment with an immune checkpoint inhibitor which is a PD-L1 inhibitor.

In some instances immunotherapy may comprise treatment with an immune checkpoint inhibitor which is a CTLA-4 inhibitor.

In some instances immunotherapy may comprise treatment with an immune checkpoint inhibitor selected from pembrolizumab, nivolumab, cemiplimab, spartalizumab, camrelizumab, sintilimab, tislelizumab, toripalimab, AMP-224, AMP-514, atezolizumab, avelumab, durvalumab, KN035, CK-301, AUNP12, CA-170, BMS-986189, ipilimumab or tremelimumab.

In some instances immunotherapy may comprise treatment with an immune checkpoint inhibitor selected from pembrolizumab, nivolumab, cemiplimab, atezolizumab, avelumab, durvalumab and ipilimumab.

In some of the claimed embodiments immunotherapy may comprise treatment with an immune checkpoint inhibitor selected from pembrolizumab and nivolumab.

In some instances immunotherapy may comprise chimeric antigen receptor T-cell therapy.

"Chimeric antigen receptor (CAR) T-cell therapy" takes some T-cells from a patient's blood, mixes them with a special virus that makes the T-cells learn how to attach to tumour cells, and then gives the cells back to the patient so they can find, attach to, and kill cancer.

In some instances immunotherapy may comprise treatment with a cytokine.

"Cytokines" are small proteins that carry messages between cells and stimulate immune cells to attack cancer.

In some instances immunotherapy may comprise treatment with an immunomodulator.

"Immunomodulators" are drugs that generally boost parts of the immune system to treat certain types of cancer.

In some instances immunotherapy may comprise treatment with a cancer vaccine.

"Cancer vaccines" are substances put into the body to start an immune response against cancer. They can be used prophylactically or to increase a body's immune response, allowing more effective treatment.

In some embodiments immunotherapy may comprise treatment with a monoclonal antibody.

"Monoclonal antibodies" (mAbs or MoAbs) are man-made versions of immune system proteins. Monoclonal antibodies can be designed to attack a very specific part of a cancer cell.

In some instances immunotherapy may comprise treatment with an oncolytic virus.

"Oncolytic virus" treatment uses viruses that have been modified in a lab to infect and kill certain tumour cells.

In some embodiments immunotherapy may comprise treatment with one immunotherapy agent.

In some embodiments immunotherapy may comprise treatment with more than one immunotherapy agent, for example, a PD-L1 or PD-1 antibody in combination with a CTLA-4 antibody.

### ATR Inhibition

An "ATR inhibitor" is any compound which attenuates the activity of the ATR enzyme *in-vitro* or *in-vivo.* ATR inhibitors may be selective or unselective, small molecules or biomolecules.

Example ATR inhibitors include AZD6738, M6620, BAY-1895344, EPT-46464, VE-821 and VX-970.

In the present invention the ATR inhibitor is AZD6738.

In some embodiments AZD6738 may be Compound (I) or a pharmaceutically acceptable salt thereof.

The term "pharmaceutically acceptable" is used to specify that an object (for example a salt, dosage form or excipient) is suitable for use in patients and/or has clinical or commercial precedence. An example list of pharmaceutically acceptable salts can be found in the "Handbook of Pharmaceutical Salts: Properties, Selection and Use", P. H. Stahl and C. G. Wermuth, editors, Weinheim/Zurich: Wiley-VCH/VFiCA, 2002 or subsequent editions.

In some embodiments AZD6738 may be Compound (I) in a salt-free form (for example in a neutral or zwitterionic form, or for example in a free base or free acid form).

In some embodiments AZD6738 may be a pharmaceutically acceptable salt of Compound (I).

A suitable pharmaceutically acceptable salt of Compound (I) is, for example, an acid-addition salt. An acid addition salt of Compound (I) may be formed by bringing the compound into contact with a suitable inorganic or organic acid under conditions known to the skilled person.

An acid addition salt may for example be formed using an inorganic acid selected from hydrochloric acid, hydrobromic acid, sulphuric acid and phosphoric acid. An acid addition salt may also be formed using an organic acid selected from citric acid, fumaric acid, maleic acid and methane sulfonic acid.

A further suitable pharmaceutically acceptable salt of Compound (I) is, for example, a salt formed within the human or animal body after administration of Compound (I) to said human or animal body.

In some embodiments AZD6738 is administered on days 1 to 21 of a 28-day cycle.

A "28-day cycle" is a single treatment period which may be continuously repeated for a given patient, or may be repeated with a treatment gap (of for example 1, 2, 3, 4, 5, 6, 7, 8, 9, 10, 11, 12, 13, 14, 30 or 60 days) between discrete cycles.

In some embodiments AZD6738 is administered on days 1 to 14 of a 28-day cycle.

In some embodiments AZD6738 is administered on days 1 to 7 of a 28-day cycle.

In some embodiments AZD6738 is administered according to a two weeks on / two weeks off (14 days on / 14 days off) schedule within a 28-day cycle.

In some embodiments AZD6738 is administered for 7 consecutive days within a 28 day cycle.

In some embodiments AZD6738 is administered for 14 consecutive days within a 28 day cycle.

In some embodiments AZD6738 is administered in a total daily dose between 30 mg and 500 mg.

In some embodiments AZD6738 is administered in a total daily dose of 40 mg, 60 mg, 80 mg, 160 mg, 240 mg, 320 mg or 480 mg.

In some embodiments AZD6738 is administered in a total daily dose of 480 mg.

In some embodiments AZD6738 is administered twice daily in 240 mg doses (i.e. in a 480 mg total daily dose, administered in two separate tranches each consisting of 50% of the total daily dose).

In some embodiments AZD6738 is administered twice daily in 240 mg doses on days 1 to 21 of a 28-day cycle.

In some embodiments AZD6738 is administered twice daily in 240 mg doses for 7 consecutive days within a 28-day cycle.

In some embodiments AZD6738 is administered twice daily in 240 mg doses on days 1 to 7 of a 28-day cycle.

In some embodiments AZD6738 is administered twice daily on a two weeks on / two weeks off schedule within a 28-day cycle.

In some embodiments AZD6738 is administered twice daily in 240mg doses for 14 consecutive days within a 28-day cycle.

In some embodiments AZD6738 is administered twice daily in 240mg doses on days 1 to 14 of a 28-day cycle.

### Cancer

"Cancer" is used synonymously with tumour and lesion in this specification. Cancer may include primary cancer as well as secondary cancers and metastases. The tumours may be detectable or non-detectable, e.g. micro metastases.

The "treatment of cancer", "treating cancer" and similar terms encompass treating an existing cancer and/or preventing cancer. In some embodiments, treatment may be conducted after one or more symptoms have developed. In other embodiments, treatment may be conducted in the absence of symptoms. For example, treatment of a susceptible individual may begin prior to the onset of symptoms (e.g. due to a history of disease and/or considering genetic or other susceptibility factors). Treatment may also be continued after symptoms have resolved, for example to present or delay their recurrence

In some embodiments the treatment of cancer or treating cancer may mean treating and preventing cancer.

In some embodiments the treatment of cancer or treating cancer may mean treating cancer.

In some embodiments the treatment of cancer or treating cancer may mean preventing cancer.

In some instances cancer may be selected from melanoma, gastric cancer, sarcoma, colon cancer, neuroendocrine cancer, hepatocellular cancer, non-small cell lung cancer, and squamous head and neck cancer.

In some instances cancer may be selected from melanoma, gastric cancer, sarcoma, colon cancer, neuroendocrine cancer and hepatocellular cancer.

In some instances cancer may be selected from melanoma, gastric cancer, non-small cell lung cancer and squamous head and neck cancer.

In some instances cancer may be selected from melanoma and gastric cancer.

In the present invention the cancer is melanoma. Mucosal and acral melanoma are known to be particularly difficult histologic subtypes of melanoma to treat. However, responses in these subtypes were observed in patients treated with the treatments described herein.

In some embodiments cancer may be cutaneous melanoma.

In some embodiments cancer may be cutaneous anal melanoma.

In some embodiments cancer may be acral melanoma.

In some embodiments cancer may be mucosal melanoma.

In some embodiments cancer may be early stage, actively progressing, advanced (for example locally advanced), invasive, metastatic and/or drug-resistant cancer.

In some embodiments cancer may be locally advanced cancer.

In some embodiments cancer may be advanced and/or metastatic cancer.

In some embodiments cancer may be locally advanced and/or metastatic cancer.

In some embodiments cancer may be metastatic cancer.

In some embodiments cancer may be metastatic melanoma.

In some embodiments cancer may be invasive cancer.

In some embodiments cancer may be Stage IV melanoma.

In some embodiments cancer may be Stage III unresectable melanoma.

### Patient Selection

In some embodiments cancer may be ATM deficient.

When a cancer is "ATM deficient", the cancer cells express less ATM protein than a normal, non-cancerous cell of the same type. For example, the cancer cells may express ≤5%, ≤10%, ≤20%, ≤30%, ≤40%, ≤50%, ≤60%, ≤70%, ≤80%, ≤90% or <100% of the total ATM protein expressed by a normal cell of the same type when analysed by IHC protein staining of the total ATM protein typically expressed by a normal cell of the same type when analysed by IHC protein staining. ATM deficient cancer cells may also comprise a biallelic deleterious mutation in their ATM gene.

In some embodiments cancer may be ARID1A deficient.

When a cancer is "ARID1A deficient", the cancer cells express less ARID1A protein than a normal, non-cancerous cell of the same type. For example, the cancer cells may express ≤5%, ≤10%, ≤20%, ≤30%, ≤40%, ≤50%, ≤60%, ≤70%, ≤80%, ≤90% or <100% of the total ARID1A protein typically expressed by a normal cell of the same type when analysed by IHC protein staining. ARID1A deficient cancer cells may also comprise a mutation in the ARID1A gene (for example a loss of function mutation such as a nonsense mutation).

In some embodiments cancer may be ATM deficient melanoma.

In some embodiments cancer may be ARID1A deficient melanoma.

In some instances the ATR inhibitor is administered to a patient as second line therapy i.e. after the patient has failed on immunotherapy.

In some instances, the ATR inhibitor is administered to a patient as third line therapy. Patients receiving the ATR inhibitor as third line therapy may have received treatment with BRAF and MEK inhibitors, such as dabrafenib and trametinib, prior to treatment with immunotherapy.

### Clinical Properties

In one embodiment there is provided the ATR inhibitor AZD6738 and paclitaxel for use in the treatment of melanoma, where the ATR inhibitor is administered to a patient who has previously received immunotherapy as defined in the claims, and treatment with the ATR inhibitor achieves an objective response rate between 10% and 50%, between 10% and 40%, between 10% and 35%, between 20% and 35%, between 25% and 40%, between 30% and 35%, greater than 10%, greater than 20%, greater than 30%, greater than 40%, greater than 50%, greater than 60% or greater than 70%.

"Objective response rate" is the percentage of patients with measurable disease at baseline and achieved at least 1 response

The "RECIST criteria" (for example the RECIST 1.1 criteria) are set out at the site https://recist.eortc.org/ and described in Eur. J. Cancer 2016, 62, Pages 132-137.

In one embodiment there is provided the ATR inhibitor AZD6738 and paclitaxel for use in the treatment of melanoma, where the ATR inhibitor is administered to a patient who has previously received immunotherapy as defined in the claims, and treatment with the ATR inhibitor achieves a clinical benefit rate between 25% and 100%, between 25% and 90%, between 40% and 80% or between 50% and 70%.

"Clinical benefit rate" is the objective response rate added to the % of patients whose best response was stable disease at the first scan i.e. who had absence of disease progression at first scan (complete response + partial response + stable disease).

In one embodiment there is provided the ATR inhibitor AZD6738 and paclitaxel for use in the treatment of melanoma, where the ATR inhibitor is administered to a patient who has previously received immunotherapy as defined in the claims, and treatment with the ATR inhibitor achieves a progression free survival of greater than 6 months, greater than 7 months, greater than 8 months, preferably greater than 9 months. In one embodiment, treatment with the ATR inhibitor achieves progression free survival of between 3 and 24 months.

"Progression free survival" or "PFS" is the length of time during and after the treatment that a patient lives without the disease worsening. PFS may be determined using the Kaplan-Meier method.

In one embodiment there is provided the ATR inhibitor AZD6738 and paclitaxel for use in the treatment of melanoma, where the ATR inhibitor is administered to a patient who has previously received immunotherapy as defined in the claims, and treatment with the ATR inhibitor achieves a duration of response of at least 2 months, at least 3 months, at least 4 months, preferably at least 5 months. In one embodiment, treatment with the ATR inhibitor achieves a duration of response of between 2 and 6 months.

"Duration of response" or "DoR" is the length of time that a tumour continues to respond to treatment without the cancer growing or spreading.

"Overall survival" or "OS" is the length of time from the start of treatment that a patient is still alive.

In one embodiment there is provided the ATR inhibitor AZD6738 and paclitaxel for use in the treatment of melanoma, where the ATR inhibitor is administered to a patient who has previously received immunotherapy, as defined in the claims, and treatment with the ATR inhibitor does not cause any serious side-effects in a cancer patient.

In some embodiments serious side-effects may be defined as grade 4 or 5 adverse events.

"Grade 4 or 5 adverse events" can be classified according to the common terminology criteria for adverse events (CTCAE).

### Combination Treatment

In one instance there is provided an ATR inhibitor for use in the treatment of cancer, where the ATR inhibitor is administered in combination with a taxane to a patient who has previously received immunotherapy.

Where the term "combination" is used it is to be understood that this refers to simultaneous, separate or sequential administration.

In some embodiments, a combination may be simultaneously, separately and/or sequentially administered.

In some embodiments, a combination may be simultaneously administered.

In some embodiments, a combination may be separately administered.

In some embodiments, a combination may be sequentially administered.

Where the administration of a combination is sequential or separate, the delay in administering the second component should not be such as to lose the beneficial effect of the combination.

A "taxane" is a diterpene compound with a taxadiene core. They can be isolated as natural products or made synthetically or semi-synthetically.

Examples of taxanes include paclitaxel, docetaxel, taxol (cremophor EL-paclitaxel), abraxane (nab-paclitaxel) and cabazitaxel.

In the present invention the taxane is paclitaxel.

In some embodiments paclitaxel is administered on days 1, 8 and 15 of a 28-day cycle.

In some embodiments paclitaxel is administered once daily on days 1, 8 and 15 of a 28-day cycle.

In some embodiments paclitaxel is administered in an amount of 80 mg/m².

In some embodiments paclitaxel is administered on days 1, 8 and 15 of a 28-day cycle in an amount of 80 mg/m².

In some embodiments paclitaxel is administered once daily on days 1, 8 and 15 of a 28-day cycle in an amount of 80 mg/m².

### Kits

Described herein is a kit comprising a pharmaceutical composition comprising an ATR inhibitor and at least one pharmaceutically acceptable excipient and instructions for the use of the pharmaceutical composition in the treatment of cancer, where the ATR inhibitor is administered to a patient who has previously received immunotherapy.

### Specific Embodiments

In one embodiment there is provided an ATR inhibitor for use in the treatment of melanoma, where the ATR inhibitor is AZD6738 which is administered in combination with paclitaxel to a patient who has previously received therapy with an immune checkpoint inhibitor selected from pembrolizumab and nivolumab.

### EXAMPLES

### Abbreviations

AE - Adverse event
AGC - Advanced Gastric Cancer
ATM - Ataxia Telangiectasia Mutated
ATR - Ataxia-Telangiectasia and Rad3-related protein
CRF - Case Report Form (electronic/paper)
CSA - Clinical Study Agreement
CSR - Clinical Study Report
CTCAE - Common Terminology Criteria for Adverse Events
CtDNA - Circulating Tumor DNA
DAE - Discontinuation of Investigational Product due to Adverse Event
DNA - Deoxyribonucleic acid
EC - Ethics Committee, synonymous to Institutional Review Board (IRB) and Independent Ethics Committee (IEC)
ECG - Electrocardiogram
GCP - Good Clinical Practice
ICH - International Conference on Harmonisation
IP - Investigational Product
LSLV - Last Subject Last Visit
OAE - Other Significant Adverse Event
PI - Principal Investigator
SAE - Serious adverse event

### Clinical Trial Protocol

**Introduction:** To determine the efficacy and dose of AZD6783 a single centre open label phase I study of AZD6738 in combination with paclitaxel in metastatic cancer patients who have failed standard chemotherapy was carried out according to the following basic protocol.

**Primary Objectives and Outcome Measures:**

| Primary Objective | Outcome Measure | |
|---|---|---|
| To evaluate the safety and tolerability of AZD6738 in combination with paclitaxel. | • AEs/SAEs | |
| | • Vital signs. | |
| | • Collection of clinical chemistry/haematology parameters. | |
| | • ECGs. The following parameters will be recorded for each ECG: date and time of ECG, heart rate (beats/min), QT (ms), QTcB (ms), sinus rhythm (yes/no). | |
| | • overall evaluation (normal/abnormal). | |
| To determine maximal tolerated dose (MTD) of AZD6738 when given in combination with fixed dose of paclitaxel in patients with refractory cancer and to evaluate toxicity profiles and dose-limiting toxicity (DLT). | • Haematological toxicities as follows: | |
| | | - Grade 4 neutropenia lasting longer than 4 consecutive days. |
| | | - Grade 3 neutropenia of any duration accompanied by fever ≥ 38.5°C and/or systemic infection. |
| | | - Grade 3 thrombocytopenia with bleeding. |
| | | - Any other confirmed haematological toxicity ≥ CTCAEv4 grade 4. |
| | • Non-haematological toxicity ≥ CTCAEv4 grade 3 including: | |
| | | - Laboratory abnormalities. |
| | | - QTc prolongation (> 500 mSec). |
| | • Any other toxicity that is greater than that at baseline, and is clinically significant and/or unacceptable, does not respond to supportive care. | |
| | • Any event, including significant dose reductions or omissions, judged to be a DLT by the SRC. | |

### Secondary Objectives and Outcome Measures:

| Secondary Objective | Outcome Measure |
|---|---|
| To obtain a preliminary assessment of the anti-tumour activity of AZD6738 in combination with paclitaxel. | • Objective Response rate and Clinical Benefit Rate (CR+PR+SD). |
| | • Overall survival (OS) and progressionfree survival (PFS) calculated by the Kaplan-Meier method. |
| To characterise the PK and pharmacodynamics (PDc) of AZD6738 following a single dose and at steady state after multiple dosing, when given orally in monotherapy and in combination with paclitaxel. | • Plasma and urine concentrations and derived pharmacokinetic parameters. |
| To obtain a preliminary assessment of AZD6738 activity in the tumour | • By evaluation of pharmacodynamic biomarker changes which may include functional ATR inhibition, ctDNA and CTCs. |

### Tertiary Objectives and Outcome Measures:

| Tertiary Objective | Outcome Measure |
|---|---|
| To evaluate biomarkers for sensitivity and resistance of AZD6738 when given in combination with fixed dose of paclitaxel. | • Retrospective sequencing to determine ATM null mutant subgroup, exploratory biomarker analyses. |
| | • To evaluate the sensitivity of AZD6738 according to ATM expression by using IHC and mutation status of other DNA repair-associated genes. |
| | • ATM status will be evaluated by IHC. Also, overall DDR genes including ATR will be analysed at the tumour. These biomarkers will be used to predict sensitivity to AZD6738 in combination with paclitaxel. |
| To investigate predictive markers for sensitivity or resistance to AZD6738 in combination with paclitaxel that may be observed in tumour (archival and/or fresh frozen) from patients treated with AZD6738 in combination with paclitaxel to determine if there are any other biomarkers that may predict sensitivity or resistance to treatment. A progression tumour specimen will also be collected for exploratory analysis to determine mechanisms of acquired resistance to treatment. | • To collect and store diagnostic and/or archival tumour samples and any fresh tumour biopsies (pre-treatment and at progression) for potential future exploratory research into factors that may influence development and/or response to AZD6738 in combination with paclitaxel. Next generation sequencing of DNA from these samples and/or other protein based exploratory analysis will be carried out. |
| To investigate predictive markers and acquired resistance to AZD6738 and/or paclitaxel that may be observed in circulating tumour DNA (ctDNA) extracted from plasma preparation of blood samples from patients treated with AZD6738 in combination with paclitaxel. | • Genomic approaches including but not limited to next generations sequencing and PCR will be used for future exploratory research into factors that can predict sensitivity to AZD6738 in combination with paclitaxel. Samples collected during treatment may be used to elucidate mechanisms of resistance to treatments. |

**Overall Study Design:** The study consisted of two parts, each evaluating the safety and tolerability of AZD6738 when given in combination with paclitaxel. An oral formulation of AZD6738 was used. The first part (Part A) was in combination with paclitaxel; the starting dose of 40 mg AZD6738 OD was escalated to reach a maximum tolerated dose in patients with advanced solid malignancies, as defined by dose-limiting toxicity. We performed a serial biopsy to explore further the biological activity of AZD6738 at selected dose(s) based on the emerging relationships between PK and PD. The second part (Part B) was a parallel PK expansion cohort with cycle 0 of AZD6738 on D1, (7-day washout for AZD6738) D8^{~}D21 monotherapy (prior to paclitaxel combination) followed by combination therapy with weekly paclitaxel from cycle 1. We made the number and doses of these cohorts flexible so that we could make adjustment as the PK data emerged. The first part was the main clinical trial. We modified to recruit the minimum or maximum number of patients depending on data generated from ongoing other studies using AZD6738. Dose escalations were conducted in each cohort independently; however, number and doses of the cohorts were to be subject to change as the PK data emerged.

**Part A:** We conducted a phase I trial for determining maximal tolerate dose (MTD) of AZD6738 when given in combination with fixed dose of paclitaxel in patients with refractory cancer. We used a rolling 6 design in this PART A. Treatment schedule was as follows. Start regimen (every 4 weeks): 40 mg OD dosing of AZD6738, 3 weeks on/ 1 week off; paclitaxel 80 mg/m2 on day 1, 8, 15 This part was in combination with paclitaxel of standard dose; starting dose of 40 mg AZD6738 OD was escalated to reach a maximum tolerated dose in metastatic solid cancer patients. Each cycle was composed of 4 weeks. The decision to dose escalate was to be made upon the assessment of the safety and tolerability data collected up until the time of study drug administration on Cycle 2, Day 1. This DLT assessment period was selected as the major toxicities leading to cessation of dose escalation in such studies (haematological, gastrointestinal, liver enzymes) were anticipated to present within this duration. Doses and/or schedules of AZD6738 were to be defined by the SRC. The cohort size of at least 3 and up to 6 patients ('rolling six design') was employed to improve the rate of accrual of patients to cohorts close to the presumed therapeutic dose by reducing the need for late replacement of patients who become non-evaluable during the DLT assessment period, whilst not compromising collection of safety data. The total number of patients depended upon the number of dose adjustments necessary. These patients had optional matched biopsies to assess the tumour for relevant pharmacodynamic biomarkers, and to explore further the tolerability, safety and pharmacokinetic activity at these doses. The cohort size of maximum 10 patients (minimum 2 patients) was employed. The matched biopsies were defined as samples at baseline, D7 and at following progression of disease (optional for this trial).

**Part B:** Parallel PK extension PART B was for evaluating pharmacokinetics. Before starting cycle 1 (4 weeks) of part B, we conducted pharmacokinetic evaluation for single dose of AZD6738 for 4 weeks, cycle 0. PART B was in combination with paclitaxel of standard dose; starting dose of 40 mg AZD6738 OD was escalated until maximum tolerated dose acquired through part A. Treatment schedule was as follows. Start regimen (every 4 weeks): 40 mg OD dosing of AZD6738, 3 weeks on/ 1 week off; paclitaxel 80 mg/m2 on day 1, 8, 15.

**Inclusion Criteria:** Patients were eligible to be included in the study only if all the following inclusion criteria and none of the exclusion criteria applied:
1. Provision of fully informed consent prior to any study specific procedures.
2. Aged at least 19 years.
3. Refractory cancer patients who had failed to standard of care chemotherapy.
4. Provision of tumour sample (from either a resection or biopsy). This criterion was optional for this study (i.e. if no biopsy sample available, it was not an exclusion criteria).
5. Patients were willing and able to comply with the protocol for the duration of the study including undergoing treatment and scheduled visits and examinations. Especially, patients must have fasted (water to drink only) from at least 2 hours prior to taking a dose to at least 1-hour post-dose for all doses. Metastatic disease or locoregionally recurrent disease which was refractory or intolerant to existing therapy(ies) known to provide clinical benefit.
6. ECOG performance status 0-1.
7. Patients must have had a life expectancy ≥ 3 months from proposed first dose date.
8. At least one measurable lesion that could be accurately assessed by imaging at baseline and following up visits.
9. Negative urine or serum pregnancy test within 28 days of study treatment, confirmed prior to treatment. Patients of child-bearing potential should have been using adequate contraceptive measures (two forms of highly reliable methods) should not have been breast feeding and must have had a negative pregnancy test prior to start of dosing, or patients must have had evidence of non-child-bearing potential by fulfilling one of the following criteria at screening:
   (a) Post-menopausal - defined as aged more than 50 years and amenorrhoeic for at least 12 months following cessation of all exogenous hormonal treatments.
   (b) Documentation of irreversible surgical sterilisation by hysterectomy, bilateral oophorectomy or bilateral salpingectomy, but not tubal ligation.
   (c) Amenorrhoeic for 12 months and serum follicle-stimulating hormone (FSH), luteinizing hormone (LH) and plasma oestradiol levels in the postmenopausal range for the institution.
10. Male patients must have been willing to use barrier contraception for the duration of the study and for one week after the last dose of study drug if the sexual partner was not of childbearing potential.

**Exclusion Criteria:** Patients did not enter the study if any of the following exclusion criteria were fulfilled:
1. More than four prior chemotherapy regimens (excluding adjuvant chemotherapy) for cancer treatment.
2. Any previous treatment with ATR inhibitors (small molecules).
3. Any previous treatment with paclitaxel (docetaxel was allowed if in physician's discretion, the tumour was not absolutely refractory to taxanes).
4. Patients with second primary cancer, except: adequately treated non-melanoma skin cancer, curatively treated in-situ cancer of the cervix, or other solid tumours curatively treated with no evidence of disease for ≤5 years.
5. Patients unable to swallow orally administered medication.
6. Treatment with any investigational product during the last 14 days before the enrolment (or a longer period depending on the defined characteristics of the agents used).
7. Patients receiving any systemic chemotherapy, radiotherapy (except for palliative reasons), within 3 weeks from the last dose prior to study treatment (or a longer period depending on the defined characteristics of the agents used). The patient could receive a stable dose of bisphosphonates or denusomab for bone metastases, before and during the study if these were started at least 4 weeks prior to treatment.
8. Concomitant use of known potent CYP3A4 inhibitors such as ketoconazole, itraconazole, ritonavir, indinavir, saquinavir, telithromycin, clarithromycin and nelfinavir. Receiving, or having received, concomitant medications, herbal supplements and/or foods that significantly modulate CYP3A4 or Pgp activity (wash out periods of two weeks, but three weeks for St. John's Wort). Note these include common azole antifungals and macrolide antibiotics.
9. Except for alopecia, any ongoing toxicities (>CTCAE grade 1) caused by previous cancer therapy.
10. Intestinal obstruction or CTCAE grade 3 or grade 4 upper GI bleeding within 4 weeks before the enrolment.
11. Resting ECG with measurable QTcF > 470 msec on 2 or more time points within a 24-hour period or family history of long QT syndrome.
12. Patients with cardiac problem as follows: uncontrolled hypertension or hypotension (BP ≥150/95 mmHg despite medical therapy, BP <100/60 mmHg or orthostatic hypotension fall in BP >20 mmHg) Left ventricular ejection fraction <55% measured by echocardiography, Atrial fibrillation with a ventricular rate >100 bpm on ECG at rest , Symptomatic heart failure (NYHA grade II-IV), Prior or current cardiomyopathy, Severe valvular heart disease, Uncontrolled angina (Canadian Cardiovascular Society grade II-IV despite medical therapy), Acute coronary syndrome within 6 months prior to starting treatment.
13. Female patients who were breast-feeding or childbearing.
14. Any evidence of severe or uncontrolled systemic disease, active infection, active bleeding diatheses or renal transplant, including any patient known to have had hepatitis B, hepatitis C or human immunodeficiency virus (HIV).
15. A diagnosis of ataxia telangiectasia.
16. Inadequate bone marrow and impaired hepatic or renal function as demonstrated by any of the following laboratory values:
   (a) Haemoglobin < 9.0 g/dL (transfusion allowed)
   (b) Absolute neutrophil count (ANC) <1.5 x 10 9 /L
   (c) White blood cells (WBC) ≤ 3 x 10 9 /L
   (d) Platelet count < 100 x 10 9 /L (transfusion allowed)
   (e) Albumin < 33g/L
   (f) Total bilirubin < 1.5 x institutional upper limit of normal (ULN)
   (g) AST (SGOT)/ALT (SGPT) > 2.5 x institutional upper limit of normal unless liver metastases were present in which case it must be > 5x ULN
   (h) Serum creatinine > 1.5 x institutional ULN
   (i) Glomerular filtration rate (GFR) < 45 mL/min, as assessed using the standard methodology at the investigating centre (i.e. Cockroft-Gault, MDRD or CKD-EPI formulae, EDTA clearance or 24 h urine collection).
   (j) Haematuria: +++ on microscopy or dipstick
   (k) INR ≥ 1.5 or other evidence of impaired hepatic synthesis function

In addition, the following were considered criteria for exclusion from the optional exploratory host genetic research:
17. Previous allogenic bone marrow transplant
18. Non-leukocyte depleted whole blood transfusion within 120 days of the date of the genetic sample collection.

**Study Treatments:** AZD6738 was administered by oral tablet in combination with paclitaxel. Patients must have fasted (water to drink only) from at least 2 hours prior to taking a dose to at least 1-hour post-dose for all doses. AZD6738 was supplied by AstraZeneca R&D as individual bottles of tablets. Combination agents were supplied by local sourcing (either directly or local supply with reimbursement). Labels were prepared in accordance with Good Manufacturing Practice and local regulatory guidelines by AstraZeneca R&D. All study drugs were kept in a secure place under appropriate storage conditions. The AZD6738 and combination agent product label on the bottle specified the appropriate storage.

The starting dose/schedule of AZD6738 was 40 mg OD daily for 21 days, followed by 7 days off treatment, in cycles of 4 weeks (28 days). The study started with PART A (paclitaxel combination) where the selected AZD6738 starting dose of 40 mg once daily was used. Patients must have fasted (water to drink only) from at least 2 hours prior to taking a dose to at least 1-hour post-dose for all doses. Before first cycle, patients received a single dose on the first day of doing (Cycle 0, Day 1) for the assessment of PK, then did not receive study drug for 6 days. And then paclitaxel was administered on Cycle 1, Day 1, Day 8 and 15 with reintroduction of once daily AZD6738 from Day 1 to Day 21 of each 28-day cycle. The starting dose/schedule of further parts was decided based on emerging safety and tolerability data.

Daily Dose, dose frequency and dosing schedule in subsequent cohorts increased or decreased in response to safety, tolerability, pharmacokinetic and emerging non-clinical data. Dose escalation and de-escalation followed the scheme below, according to the following logic:
(a) If no dose-limiting toxicity (DLT) is observed in a cohort of 3 evaluable patients then dose escalation may occur. Dose increases will be permitted after review of data from a minimum of 3 evaluable patients has been performed.
(b) If one patient experiences a DLT in a group of 3 or more evaluable patients then the cohort will be expanded to include 6 evaluable patients. If only one DLT is observed in the complete cohort of 6 evaluable patients then dose escalation may occur.
(c) If 2 or more patients experience a DLT in a group of up to 6 evaluable patients, irrespective of the number of patients enrolled, the dose will be considered not tolerated and recruitment to the cohort and dose escalation will cease. A lower, intermediate dose (de-escalation) may be considered in order to better define the MTD.

There was no minimum time period required between completion of dosing in the last evaluable patient from one cohort and the start of dosing in the subsequent cohort. There was no intra-patient dose escalations. After each dose level during the dose escalation phase of the study, the investigators evaluated the safety and tolerability and pharmacokinetics of AZD6738 to decide the next dose and/or schedule.

Based on emerging data, any decisions made by the SRC to change the next cohort were as follows:
(a) Modify the dose, daily dose frequency or schedule of AZD6738*, to either increase, maintain or reduce the exposure of AZD6738 over a given cycle in response to emerging tolerability data:
(b) Escalate dose of AZD6738 if the dose is deemed tolerated by the investigators; or
(c) De-escalate the dose of AZD6738 (for doses above 20mg), either to a previous lower dose level (up to a maximum of 6 evaluable patients) or to an intermediate lower dose level, at a particular schedule, if the dose is deemed non-tolerated by the investigators; or
(d) Add additional days to time off treatment with AZD6738 following the dose of paclitaxel with corresponding reduction of treatment days with AZD 6738, if the current schedule is deemed to be non-tolerated by the investigators; or
(e) Increase the time off treatment with AZD6738 prior to the first cycle of paclitaxel based on emerging data;
(f) Increase the daily dose frequency of AZD6738 dependent upon emerging data; or
(g) Decrease the daily dose frequency of AZD6738 dependent upon emerging data

Changes to only 2 of the following parameters may occur between successive cohorts:
(h) AZD6738 dose.
(i) AZD6738 daily dose frequency.
(j) AZD6738 schedule.

However, no more than a 2-fold increase in total cycle dose or predicted Cmax compared to the prior maximum tolerated total cycle dose was to occur. Paclitaxel schedule and dose was fixed.

**Dose Limiting Toxicity (DLT):** A dose was considered non-tolerated and dose escalation ceased if 2 or more of up to 6 evaluable patients experienced a DLT at a dose level. Once the non-tolerated dose was defined the MTD was to be confirmed at the previous dose-level below the non-tolerated dose or a dose between the non-tolerated dose and the last tolerated dose was to be investigated. Six evaluable patients were required to determine the MTD.

A DLT was defined as any toxicity during the periods of Cycle 0 (monotherapy) and Cycle 1 (combination) (i.e., from dosing on Cycle 0, Day 1 until the last day of dosing in Cycle 1), which included:
(a) Haematological toxicities as follows:
   - Grade 4 neutropenia (ANC < 500 cells/mm3) lasting longer than 4 consecutive days
   - Grade 3 neutropenia (ANC ≥500 to <1000 cells/mm3) of any duration accompanied by fever ≥ 38.5°C and/or systemic infection.
   - Grade 3 thrombocytopenia (25,000 to <50,000/mm3) with bleeding.
   - Any other confirmed haematological toxicity ≥ CTCAEv4 grade 4 (a repeat may have been required for confirmation of an isolated abnormality in the absence of clinical signs, symptoms or other abnormal investigations, i.e. a suspected spurious value).
(b) Non-haematological toxicity ≥ CTCAEv4 grade 3 including:
   - Laboratory abnormalities (a repeat may have been required for confirmation of an isolated abnormality in the absence of clinical signs, symptoms or other abnormal investigations, i.e. a suspected spurious value).
   - QTc prolongation (> 500 msec).
(c) Any other toxicity that was greater than that at baseline, and was clinically significant and/or unacceptable, did not respond to supportive care.
(d) Any event, including significant dose reductions or omissions, judged to be a DLT by the SRC.
(e) A DLT excluded:
   - Alopecia of any grade.
   - Inadequately treated grade 3 nausea and/or vomiting and grade 3 diarrhoea (any such patients should have received optimal antiemetic and/or antidiarrheal prophylaxis and/or treatment).
   - Any toxicity clearly unrelated to AZD6738/carboplatin combination e.g. solely related to the disease or disease-related process under investigation.

**Efficacy Assessment:** This study assessed the efficacy of AZD6738 when given in combination with paclitaxel in patients with metastatic solid cancer. RECIST 1.1 criteria was used to assess patient response to treatment by determining PFS and ORR. The RECIST 1.1 guidelines for measurable, non-measurable, target and non-target lesions and the objective tumour response criteria (complete response, partial response, stable disease or progression of disease). The methods of assessment of tumour burden used at baseline, CT or MRI scans of chest, abdomen and pelvis were to be used at each subsequent follow-up assessment. Following the baseline assessment, efficacy for all patients was to be assessed by objective tumour assessments every 8 weeks relative to date of first dose, until week 40, at which time assessments were to be carried out every 12 weeks until objective disease progression as defined by RECIST 1.1.

If a patient discontinued treatment (and/or received a subsequent cancer therapy) prior to progression, then the patient was to continue to be followed until objective disease progression as defined by RECIST 1.1.

Categorisation of objective tumour response assessment was based on the RECIST 1.1 criteria of response: CR (complete response), PR (partial response), SD (stable disease) and PD (progression of disease). Target lesion (TL) progression was calculated in comparison to when the tumour burden was at a minimum (i.e. smallest sum of diameters previously recorded on study). In the absence of progression, tumour response (CR, PR, SD) was calculated in comparison to the baseline tumour measurements obtained before starting treatment.

For any patients with non-measurable disease only at baseline, categorisation of objective tumour response assessment was based on the RECIST 1.1 criteria of response: CR, PD and Non-CR/Non-PD. If the investigator was in doubt as to whether progression had occurred, particularly with response to NTL (non-target lesion) or the appearance of a new lesion, it was advisable to continue treatment until the next scheduled assessment or sooner if clinically indicated and the patient's status to be reassessed. If repeat scans confirmed progression, then the date of the initial scan was to be declared as the date of progression.

Any other sites at which new disease was suspected was also to be appropriately imaged. If an unscheduled assessment was performed and the patient had not progressed, every attempt was to be made to perform the subsequent assessments at their scheduled visits. To achieve 'unequivocal progression' on the basis of non-target disease, there must have been an overall level of substantial worsening in non-target disease such that, even in presence of SD or PR in target disease, the overall tumour burden had increased sufficiently to merit discontinuation of therapy. A modest 'increase' in the size of one or more non-target lesions is usually not enough to quality for unequivocal progression status.

Following progression, patients were to continue to be followed up for survival weeks as outlined in the Study Schedule (see Table 1). It was important to follow the assessment schedule as closely as possible.

**Safety and Clinical Assessments:** The safety and tolerability of AZD6783 was the primary objective of this study. Related outcome measures were DLTs, AEs/SAEs, vital signs, clinical chemistry/haematology, and ECGs. Laboratory safety assessments included:

Full haematology assessments:
(a) Haemoglobin.
(b) Red blood cells (RBC).
(c) Platelets.
(d) Mean cell volume (MCV).
(e) Mean cell haemoglobin concentration (MCHC).
(f) Mean cell haemoglobin (MCH).
(g) WBC.
(h) Absolute differential white cell count (neutrophils, lymphocytes, monocytes, eosinophils and basophils) and absolute neutrophil count or segmented neutrophil count and Band forms were to be performed at each visit and when clinically indicated. If absolute differentials not available % differentials were to be provided.
(i) Coagulation:
   - Activated partial thromboplastin time (APTT) was to be performed at baseline and if clinically indicated
   - International normalised ratio (INR) was to be performed at baseline and if clinically indicated unless the patient is receiving warfarin. Patients taking warfarin were able to participate in this study; however, it was recommended that prothrombin time (INR and APTT) be monitored carefully at least once per week for the first month, then monthly if the INR was stable.
(j) Biochemistry assessments for safety:
   - Sodium
   - Potassium
   - Calcium
   - Magnesium
   - Creatinine
   - Total bilirubin
   - Gamma glutamyl transferase (GGT)
   - ALP
   - AST
   - ALT
   - Urea or blood urea nitrogen (BUN)
   - Total protein
   - Albumin
   - Lactic dehydrogenase (LDH).

Urinalysis: urinalysis was to be performed at screening and if clinically indicated. Microscopic analysis was to be performed by the hospital's local laboratory if required. If being assessed within 7 days before first dose and meets the stated eligibility criteria (if applicable), it need not have been repeated on Day 1 of Cycle 1, unless investigator believed that it was likely to have changed significantly.

Physical examination: A complete physical examinations was performed including an assessment of the following: general appearance, respiratory, cardiovascular, abdomen, skin, head and neck (including ears, eyes, nose and throat), lymph nodes, thyroid, abdomen, musculo-skeletal (including spine and extremities) and neurological systems. Performance status was assessed using the ECOG scale at screening and as outlined in the study schedule. The same observer was to assess performance status each time. If being assessed within 7 days before first dose and meets the stated eligibility criteria (if applicable), it did not need to be repeated on Day 1 of Cycle 1, unless investigator believed that it was likely to have changed significantly.

ECG: ECGs were required within 7 days prior to starting study treatment, when clinically indicated and at the follow up visit after patient had discontinued study medication. Twelve-lead ECGs were obtained after the patient had been rested in a supine position for at least 5 minutes in each case. All 12-lead ECGs were to be recorded while the patient was in the supine position. The investigator or designated physician was to review the paper copies of each of the timed 12-lead ECGs on each of the study days when they were collected. ECGs were to be recorded at 25 mm/sec. All ECGs were to be assessed by the investigator as to whether they were clinically significantly abnormal / not clinically significantly abnormal. If there was a clinically significant abnormal finding, the investigator was to record it as an AE on the CRF. A copy of the ECG indicating the study number and E-code was to be included in the study file.

Vital signs: Height was to be assessed at screening only. Weight was to be assessed at screening and as clinically indicated at any other time. Any changes in vital signs were to be recorded as an AE, if applicable. Supine BP and pulse rate were to be measured using a semi-automatic BP recording device with an appropriate cuff size, after patient had rested for at least 10 minutes and was to be assessed on Day 1 of Cycle 0, and D 1, D8, D15 Cycle 1 and then to be performed every cycle. (within a window of +/- 7 days of the scheduled date). Orthostatic hypotension was monitored. The date and time of collection and measurement was recorded on the appropriate CRF. Body temperature was measured in degrees Celsius using an automated thermometer at screening and as clinically indicated at any other time. The date of collection and measurement was to be recorded on the appropriate CRF.

Other Safety Assessments: two pregnancy tests on blood or urine samples was to be performed for any pre-menopausal women of childbearing potential one within 28 days prior to the start of study treatment and the other on Day 1 of the study prior to commencing treatment. Tests were to be performed by the hospital's local laboratory. If results were positive the patient was ineligible/discontinued from the study.

**Concomitant Medications:** any medications (other than those excluded by the clinical trial protocol) that were considered necessary for the subject's welfare and would not interfere with the trial medication were to be given at the investigator's discretion. The investigator was to record all concomitant medication taken by subject during the trial, from the date of signature of informed consent, in the appropriate section of the CRF.

The following were not permitted during the trial:
(a) Prophylactic granulocyte colony stimulating factor or granulocyte macrophage colony stimulating factor.
(b) Concomitant use of known potent CYP3A4 inhibitors such as ketoconazole, itraconazole, ritonavir, indinavir, saquinavir, telithromycin, clarithromycin and nelfinavir.
(c) To avoid potential reductions in exposure due to drug interactions, the following CYP3A4 inducers were to be avoided: Phenytoin, rifampicin, rifapentine, rifabutin, carbamazepine, phenobarbitone, nevirapine, modafinil and St John's Wort.
(d) Traditional Chinese Medicine with an approved anticancer indication.

**Pharmacokinetics:** in cycle 0 of Part A, B, venous blood samples (2 mL) for determination of concentrations of AZD6738 and relevant active metabolites in plasma were taken. The date and time of collection of each sample was recorded. There was to be a minimum of 6 patients recruited per cohort at designated PK sites in cycle 0 of Part A, parallel PK extension study. In cycle 1 of Part A, B, venous blood samples (4 mL) for determination of concentrations of AZD6738 and AZ13368982 (active metabolite) or paclitaxel and characterisation of metabolites in plasma were taken at the times presented. The date and time of collection of each sample was to be recorded. Samples were to be split, one for AZD6738 and relevant active metabolite and one for the metabolite identification work.

Samples for determination of AZD6738 and the active metabolite concentrations in plasma and AZD6738 concentrations in urine and paclitaxel concentrations in plasma were analysed using appropriate bioanalytical methods. All samples still within the known stability of the analytes of interest (i.e. AZD6738 or paclitaxel) at the time of receipt by the bioanalytical laboratory were analysed. In addition, the pharmacokinetic samples may have been subjected to further analyses in order to further investigate the presence and/or identity of drug metabolites. Any results from such analyses were to be reported separately from the Clinical Study Report.

**Pharmacodynamics:** Pharmacodynamic (PDc) biomarkers was a key secondary objective of this study and was used to inform the extent and duration of ATR target inhibition following treatment with AZD6738 in patients with AGC. PDc biomarker analyses was also used to obtain a preliminary assessment of the AZD6738 PK-PD relationship in patients and determine any retrospective correlations with ATM status and response to AZD6738 treatment.

**Biomarkers:** The collection of blood based PDc biomarker samples was mandatory for this phase I trial. Peripheral blood samples were collected and analysed for downstream analysis of ATR pathway biomarkers that may have included but were not limited to γ-H2AX, pCHK, pATR in purified peripheral blood mononuclear cells (PBMC) samples. Peripheral blood (10 mL) was collected as detailed in the study plan +/- 1 day should the visit have occurred at the weekend. Additional details of blood, archival and fresh frozen tissue collection, shipping and storage of samples for biomarker assays can be found in the laboratory manual.

Formalin fixed tumour tissue embedded in paraffin blocks were to be requested for all patients. If baseline biopsy samples could also be collected, retrieval of the archival diagnostic tumour material was still highly encouraged, to provide data on how the tumour had evolved since diagnosis. Archival samples from either primary or metastatic tumour were accepted but tissue from the primary tumour was preferred. Tissue from the most recent biopsy was preferred where a patient had archival tissue samples from multiple time points. Freshly prepared unstained slides (minimum 10, preferably 20), 4 micro sections from the archival tumour block were accepted if tumour blocks could not be submitted, however tumour tissue blocks were preferred.

A 10 mL peripheral blood sample was collected to provide plasma for circulating tumour DNA, for the analysis of predictive biomarkers and to interrogate changes in genetic alterations and potential mechanisms of resistance to AZD6738 treatment. Blood samples for ctDNA were to be collected at the following timepoints: pre-treatment Cycle 0, Day 1, on Cycle 1, Day 1 and every cycle, Day 1 and at discontinuation of therapy.

### Clinical Trial Results

In order for a patient to be evaluable for DLT assessment, they must have received at least 75% of the specified dose of ceralasertib during cycle 1, or experienced a DLT during the same period. Tumor responses were evaluated every 2 cycles for the first 10 cycles, followed by every 3 cycles until progression using computed tomography according to the RECIST 1.1 criteria.

57 patients (33 melanoma, 15 gastric cancer (GC), 4 sarcoma, 3 colon cancer, 1 neuroendocrine and 1 hepatocellular cancer) were enrolled in a clinical trial according to the above protocol, comprising 7 dose cohorts ranging 40mg OD to 240 mg BID. One dose-limiting toxicity (DLT) of neutropenic fever occurred in each cohort of n=6 evaluable patients at AZD6738 160 mg BD and 240 mg BD days 1-14. Per protocol, the maximum tolerated dose of AZD6738 was 240 mg BID days 1-14.

From an initial data set, the most common toxicities (all causality, all grade) were anorexia (n=15, 26%), nausea (n=15, 26%), leukopenia (n=12, 21%) and anaemia (n=11, 19%). 51 patients were evaluable for efficacy, with the following results being observed: 1 complete response (1.9%, melanoma), 12 confirmed partial responses (23.5%; 2 gastric cancer, 10 melanoma all of which were post-immunotherapy), 18 stable disease (35.3%) and 20 disease progression (39.2%). The overall confirmed response rate from the dose escalation was 25.5%. The confirmed response rate (based on clinical investigator assessment) observed for melanoma subjects specifically was 10/30 patients, or 33.3%.

From an updated data set, the most common treatment-emergent adverse events (all causality, all grades) were anaemia (n= 27, 47%) and neutropenia, including neutrophil count decreased (n=24, 42%). A summary of adverse events is provided in Table 3. In the efficacy set (of 57 patients who received at least one dose of ceralasertib or paclitaxel), there was 1 complete response (CR) (1.8 %, melanoma), 12 confirmed partial responses (PRs) (12/57 [21.1%]; 2 gastric, 10 melanoma, all of the melanoma cases having received prior immunotherapy), 18 patients with a best response of stable disease (SD, 31.6%), 22 patients with progressive disease (PD) and 4 were non-evaluable. At the data cut-off, three patients were still receiving study drugs. In the subgroup of patients with melanoma (n = 33), the ORR was 33.3 % (95% Cl, 18.0 - 51.8) and the DCR was 60.6% (95% Cl 42.1 - 77.1%). The median PFS for the melanoma patients was 3.60 months (95% Cl 2.00 - 5.78), the median duration of response was 9.9 months (95% CI 3.7 - 23.2) and mOS was 7.4 months (95% Cl 5.7 - 11.9). Responses did not appear related to the baseline level of LDH since responses were observed in patients with high baseline LDH, which is a negative prognostic indicator in melanoma, suggesting a benefit even in this group with poor therapeutic outcomes (there were 8 responders from 28 with LDH >ULN). Responses also did not appear related to PD-L1 expression (there were 6 responders from 12 with PD-L1 expression ≤5%). Furthermore, responses were observed across all histological subtypes of melanoma, including in 3 of 11 cutaneous, 3 of 10 acral, and 5 of 11 mucosal. Two of the melanoma patients who responded to study treatment had responses to prior PD-1 immunotherapy; 6 had SD as the best response and 3 had PD as the best response.

Genomic analysis of baseline plasma (27 patients) revealed enrichment of NF1 somatic mutations and activating NRAS mutations amongst melanoma patients (6/18 and 4/18, respectively). Cyclical changes in interleukin-12 levels were observed in three patients with disease control which could reflect an immunological component to the mechanism of response.

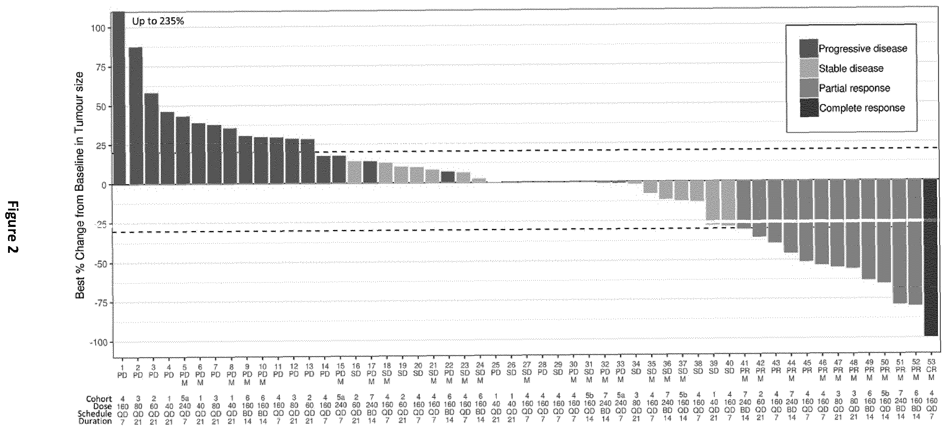

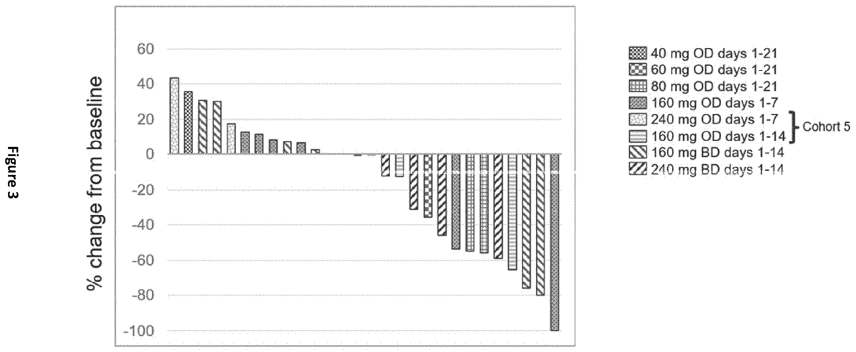

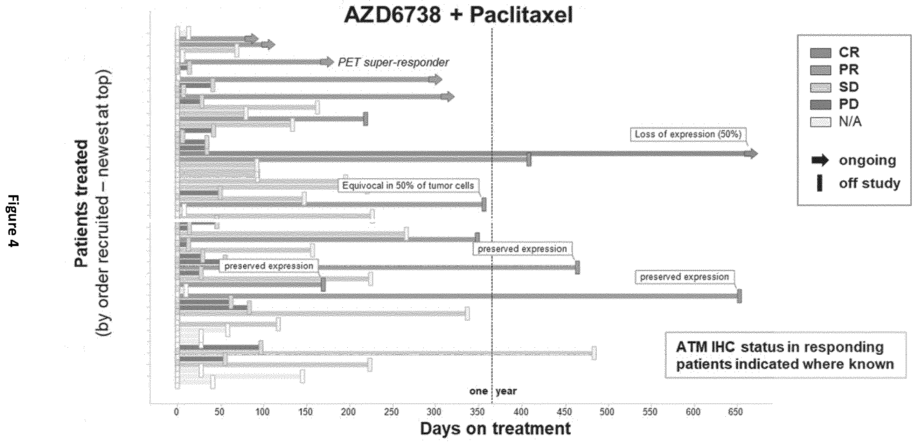

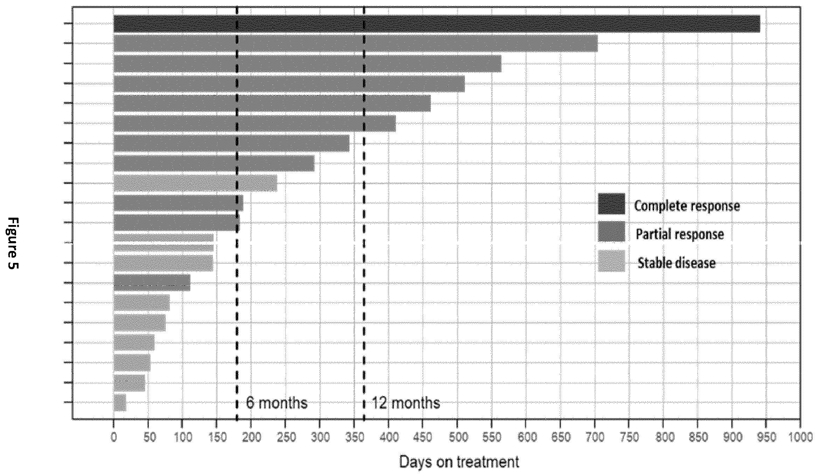

Figures 2, 3, 4 and 5 present a selection of the clinical trial data in graphical form.

**Table 1A: Clinical characteristics of the melanoma patients receiving AZD6738 (ceralasertib) and paclitaxel**

| **Melanoma patients (N= 33)** | | | |
|---|---|---|---|
| Prior anti-PD(L)1/ | | | 33 (100.0) |
| Prior anti-CTLA4 therapy | | | 0 |
| Prior RAF inhibitor | | | 6 (18.1) |
| Prior lines of chemotherapy | | | |
| | 1 regimen | | 14 (42.4) |
| | 2 regimens | | 19 (57.6) |
| LDH expression | | | |
| | | ≤ULN | 5 (15.2) |
| | | >ULN to ≤2x ULN | 10 (30.3) |
| | | >2x ULN | 18 (54.5) |
| Best response to prior anti-PD1/L1 | | | |
| | | CR or PR | 4 (12.1) |
| | | SD | 16 (48.5) |
| | | PD | 13 (39.4) |
| BRAF gene | | | 3 (9.0) |
| NRAS gene | | | 6 (18.2) |
| Brain metastasis | | | 3 (9.0) |

| Melanoma subtypes | | | |
|---|---|---|---|
| | | Acral | 10 (30.3) |
| | | Cutaneous | 11 (33.3) |
| | | Mucosal | 11 (33.3) |
| | | Unknown | 1 (3.1) |
| | | Stage IV melanoma | 33 (100.0) |

**Table 1B: Clinical characteristics of the melanoma patient responders receiving AZD6738 (ceralasertib) and paclitaxel**

| | **Ceralasertib + paclitaxel (total n=33; responders n=11)** |
|---|---|
| Responses in IO refractory patients | 7 had SD as best response on prior IO |
| | 4 had PD as best response on prior IO |
| Type of resistance to prior PD-(L)1 | Primary n=19 of which 5 responses (26%) |
| | Secondary n=7 of which 4 responses (57%) |
| | After discontinuation n=5 of which 1 response (20%) |
| | Non-evaluable n=2 of which 1 response (50%), (insufficient drug exposure) |
| Responses in all 3 melanoma subtypes | 3 Cutaneous |
| | 2 Acral |
| | 5 Mucosal |
| | 1 Unknown |
| Responses in patients with high baseline LDH, a poor prognostic indicator | 3 with ≤ULN |
| | 5 with >ULN and ≤2xULN |
| | 3 with >2xULN |
| Responses in patients with low PD-L1 expression (investigator assessed) | 6/11 had PD-L1 expression 0-5% |
| | 5/11 unknown |
| Brain mets | None of the responders had brain mets |
| Liver mets | 4/17 (24%) |
| No clear association with BRAF, NRAS or KRAS status | 1 responder had BRAF mutation |

| | |
|---|---|
| IO: immunotherapy; SD: stable disease; PD: progressive disease; PR: partial response; LDH: lactate dehydrogenase; ULN: upper limit of normal; primary and secondary resistance defined according to SITC recommendations. | |

**Table 2: Primary and secondary resistance to prior immunotherapy in melanoma patients and response to AZD6738 (ceralasertib) and paclitaxel**

| | **Primary** | **Secondary** | **Early relapse** | **PD after discontinuation** | **NE (not evaluable)** |
|---|---|---|---|---|---|
| **Ceralasertib + paclitaxel** | **19 (58%)** | **7 (21%)** | **N/A** | **5 (15%)** | **2 (6%)** |
| ORR | 5 (26%) | 4 (57%) | | | |
| DCR | 11 (58%) | 4 (57%) | | | |
| PFS | 2.2 mo | 10.9 mo | | | |
| OS | 7.2 mo | 25.5 mo | | | |

| | | | | | |
|---|---|---|---|---|---|
| ORR: objective response rate; DCR: disease control rate; PFS: progression free survival; OS: overall survival; primary and secondary resistance defined according to SITC recommendations. | | | | | |

**Table 3: Treatment-emergent adverse events occurring in >10% of patients in the safety analysis set by CTCAE grade**

| | **All patients and doses (N=57)** | | **160mg BD melanoma (N=6)** | | **240mg BD melanoma (N=9)** | |
|---|---|---|---|---|---|---|
| | **Any Grade n(%)** | **Grade ≥ 3 n(%)** | **Any Grade n(%)** | **Grade ≥ 3 n(%)** | **Any grade n(%)** | **Grade≥ 3 n(%)** |
| **Patients with any AE** | 57 (100%) | 28 (49.1%) | 6 (100%) | 4 (67%) | 5 (56%) | 5 (56%) |
| **Anaemia** | 27 (47%) | 13 (23%) | 5 (83.3%) | 3 (50%) | 2 (22.2%) | 2 (22.2%) |
| **Neutropenia and neutrophil count decreased** | 24 (42%) | 17 (30%) | 4 (83.3%) | 4 (67%) | 5 (56%) | 5 (56%) |
| **Anorexia** | 17 (30%) | 0 | 2 (33.3%) | 0 | 2 (22.2%) | 0 |
| **Nausea** | 17 (30%) | 1 (2%) | 2 (33.3%) | 0 | 3 (33.3%) | 0 |
| **Alopecia** | 15 (26%) | 0 | 2 (33.3%) | 0 | 0 | 0 |
| **Fatigue** | 10 (17%) | 0 | 1 (17%) | 0 | 3 (33.3%) | 0 |
| **Platelet count decreased & thrombocytopenia** | 7 (12%) | 5 (9%) | 1 (17%) | 1 (17%) | 4 (44.4%) | 3 (33.3%) |
| **Pruritus** | 7 (12%) | 0 | 2 (33.3%) | 0 | 4 (44.4) | 0 |
| **Vomiting** | 7 (12%) | 1 (2%) | 1 (17%) | 0 | 2 (22.2%) | 0 |
| **Rash** | 7 (12%) | 0 | 2 (33.3%) | 0 | 1 (11.1%) | 0 |

## Claims

1. An ATR inhibitor for use in the treatment of melanoma in a patient, wherein said treatment comprises the separate, sequential or simultaneous administration of:
a. the ATR inhibitor AZD6738, and
b. paclitaxel,
to said patient, wherein the patient has previously received immunotherapy comprising treatment with a PD-1 inhibitor or a PD-L1 inhibitor.

2. An ATR inhibitor for use as claimed in claim 1, where AZD6738 is administered on days 1 to 14 of a 28-day cycle.

3. An ATR inhibitor for use as claimed in claim 1 or claim 2, where the total daily dose of AZD6738 is between 30 mg and 500 mg.

4. An ATR inhibitor for use as claimed in any of the preceding claims, where AZD6738 is administered twice daily in 240 mg doses.

5. An ATR inhibitor for use as claimed in any of the preceding claims, where paclitaxel is administered on days 1, 8 and 15 of a 28-day cycle.

6. An ATR inhibitor for use as claimed in any of the preceding claims, where paclitaxel is administered in an amount of 80 mg/m².

7. An ATR inhibitor for use as claimed in any of the preceding claims, where the melanoma is cutaneous melanoma.

8. An ATR inhibitor for use as claimed in any one of claims 1 to 6, where the melanoma is acral melanoma.

9. An ATR inhibitor for use as claimed in any one of claims 1 to 6, where the melanoma is mucosal melanoma.

10. An ATR inhibitor for use according to any of the preceding claims, where the patient has primary resistance to immunotherapy.

11. An ATR inhibitor for use according to any one of claims 1 to 9, where the patient has acquired resistance to immunotherapy.

12. An ATR inhibitor for use as claimed in any one of the preceding claims, where the patient has previously received immunotherapy comprising treatment with a PD-1 inhibitor.

13. An ATR inhibitor for use as claimed in claim 12, where the PD-1 inhibitor is selected from the group consisting of pembrolizumab and nivolumab.

14. An ATR inhibitor for use as claimed in any one of claims 1 to 11, where the patient has previously received immunotherapy comprising treatment with a PD-L1 inhibitor.

15. An ATR inhibitor for use according to claim 14, where the PD-L1 inhibitor is selected from the group consisting of atezolizumab, avelumab, durvalumab, KN035, CK-301, AUNP12, CA-170 and BMS-986189.

## Patentansprüche

1. ATR-Inhibitor zur Verwendung bei der Behandlung von Melanom bei einem Patienten, wobei die Behandlung die getrennte, sequenzielle oder gleichzeitige Verabreichung von:
a. dem ATR-Inhibitor AZD6738, und
b. Paclitaxel,
an den Patienten umfasst, wobei der Patient zuvor eine Immuntherapie erhalten hat, umfassend eine Behandlung mit einem PD-1-Inhibitor oder einem PD-L1-Inhibitor.

2. ATR-Inhibitor zur Verwendung nach Anspruch 1, wobei AZD6738 an den Tagen 1 bis 14 eines 28-Tage-Zyklus verabreicht wird.

3. ATR-Inhibitor zur Verwendung nach Anspruch 1 oder 2, wobei die tägliche Gesamtdosis von AZD6738 zwischen 30 mg und 500 mg liegt.

4. ATR-Inhibitor zur Verwendung nach einem der vorstehenden Ansprüche, wobei AZD6738 zweimal täglich in 240 mg Dosen verabreicht wird.

5. ATR-Inhibitor zur Verwendung nach einem der vorstehenden Ansprüche, wobei Paclitaxel an den Tagen 1, 8 und 15 eines 28-Tage-Zyklus verabreicht wird.

6. ATR-Inhibitor zur Verwendung nach einem der vorstehenden Ansprüche, wobei Paclitaxel in einer Menge von 80 mg/m² verabreicht wird.

7. ATR-Inhibitor zur Verwendung nach einem der vorstehenden Ansprüche, wobei das Melanom ein kutanes Melanom ist.

8. ATR-Inhibitor zur Verwendung nach einem der Ansprüche 1 bis 6, wobei das Melanom ein akrales Melanom ist.

9. ATR-Inhibitor zur Verwendung nach einem der Ansprüche 1 bis 6, wobei das Melanom ein Schleimhautmelanom ist.

10. ATR-Inhibitor zur Verwendung nach einem der vorstehenden Ansprüche, wobei der Patient eine primäre Resistenz gegen Immuntherapie aufweist.

11. ATR-Inhibitor zur Verwendung nach einem der Ansprüche 1 bis 9, wobei der Patient eine erworbene Resistenz gegen Immuntherapie aufweist.

12. ATR-Inhibitor zur Verwendung nach einem der vorstehenden Ansprüche, wobei der Patient zuvor eine Immuntherapie erhalten hat, umfassend die Behandlung mit einem PD-1-Inhibitor.

13. ATR-Inhibitor zur Verwendung nach Anspruch 12, wobei der PD-1-Inhibitor aus der Gruppe ausgewählt ist, bestehend aus Pembrolizumab und Nivolumab.

14. ATR-Inhibitor zur Verwendung nach einem der Ansprüche 1 bis 11, wobei der Patient zuvor eine Immuntherapie erhalten hat, umfassend die Behandlung mit einem PD-L1-Inhibitor.

15. ATR-Inhibitor zur Verwendung nach Anspruch 14, wobei der PD-L1-Inhibitor aus der Gruppe ausgewählt ist, bestehend aus Atezolizumab, Avelumab, Durvalumab, KN035, CK-301, AUNP12, CA-170 und BMS-986189.

## Revendications

1. Inhibiteur d'ATR pour utilisation dans le traitement d'un mélanome chez un patient, dans lequel ledit traitement comprend l'administration séparée, séquentielle ou simultanée de :
a. l'inhibiteur d'ATR AZD6738, et
b. paclitaxel,
audit patient, dans lequel le patient a précédemment reçu une immunothérapie comprenant un traitement avec un inhibiteur de PD-1 ou un inhibiteur de PD-L1.

2. Inhibiteur d'ATR pour utilisation selon la revendication 1, où AZD6738 est administré aux jours 1 à 14 d'un cycle de 28 jours.

3. Inhibiteur d'ATR pour utilisation selon la revendication 1 ou la revendication 2, où la dose journalière totale d'AZD6738 est comprise entre 30 mg et 500 mg.

4. Inhibiteur d'ATR pour utilisation selon l'une quelconque des revendications précédentes, où AZD6738 est administré deux fois par jour en doses de 240 mg.

5. Inhibiteur d'ATR pour utilisation selon l'une quelconque des revendications précédentes, où du paclitaxel est administré aux jours 1, 8 et 15 d'un cycle de 28 jours.

6. Inhibiteur d'ATR pour utilisation selon l'une quelconque des revendications précédentes, où du paclitaxel est administré en une quantité de 80 mg/m².

7. Inhibiteur d'ATR pour utilisation selon l'une quelconque des revendications précédentes, où le mélanome est un mélanome cutané.

8. Inhibiteur d'ATR pour utilisation selon l'une quelconque des revendications 1 à 6, où le mélanome est un mélanome acral.

9. Inhibiteur d'ATR pour utilisation selon l'une quelconque des revendications 1 à 6, où le mélanome est un mélanome muqueux.

10. Inhibiteur d'ATR pour utilisation selon l'une quelconque des revendications précédentes, où le patient a une résistance primaire à une immunothérapie.

11. Inhibiteur d'ATR pour utilisation selon l'une quelconque des revendications 1 à 9, où le patient a acquis une résistance à une immunothérapie.

12. Inhibiteur d'ATR pour utilisation selon l'une quelconque des revendications précédentes, où le patient a précédemment reçu une immunothérapie comprenant un traitement avec un inhibiteur de PD-1.

13. Inhibiteur d'ATR pour utilisation selon la revendication 12, où l'inhibiteur de PD-1 est choisi dans le groupe constitué de pembrolizumab et de nivolumab.

14. Inhibiteur d'ATR pour utilisation selon l'une quelconque des revendications 1 à 11, où le patient a précédemment reçu une immunothérapie comprenant un traitement avec un inhibiteur de PD-L1.

15. Inhibiteur d'ATR pour utilisation selon la revendication 14, où l'inhibiteur de PD-L1 est choisi dans le groupe constitué d'atezolizumab, d'avelumab, de durvalumab, de KN035, de CK-301, d'AUNP12, de CA-170 et de BMS-986189.
